(19) Europäisches Patentamt
European Patent Office
Office européen des brevets

(11) Publication number: **0 287 215 B1**

(12) **EUROPEAN PATENT SPECIFICATION**

(45) Date of publication of patent specification: **17.06.92**

(51) Int. Cl.[5]: **C07H 19/073**, A61K 31/70

(21) Application number: **88302233.7**

(22) Date of filing: **15.03.88**

(54) **Therapeutic nucleosides.**

(30) Priority: **16.03.87 GB 8706176**
**28.12.87 US 138808**

(43) Date of publication of application:
**19.10.88 Bulletin 88/42**

(45) Publication of the grant of the patent:
**17.06.92 Bulletin 92/25**

(84) Designated Contracting States:
**AT BE CH DE ES FR GB GR IT LI LU NL SE**

(56) References cited:
**EP-A- 0 196 185**
**EP-A- 0 199 451**

**PROCEEDINGS OF THE NATIONAL ACADEMY OF SCIENCES, vol. 82, no. 20, 1985, USA H.MITSUYA et al. "3'-Azido- - 3'-deoxythylmidine (BW A5oqU): An antiviral agent that inhibits the infectivity and cytopathic effect of human T-lymphotro- pic virus type III/lympha- denopathy-associated virus in vitro" pages 7096-7100**

**THE JOURNAL OF ORGANIC CHEMISTRY, vol. 29, 1964 HORWITZ et al. "Nucleosides. V. The monomezylates of 1-(2'deoxy beta-D-lyxofura- nosyl)-thymine" pages 2076-2078**

(73) Proprietor: **THE WELLCOME FOUNDATION LIMITED**
**Unicorn House 160 Euston Road**
**London NW1 2BP(GB)**

(72) Inventor: **Rideout, Janet Litster**
**3101 Morningside Drive**
**Raleigh North Carolina 27607(US)**
Inventor: **Freeman, George Andrew**
**107 Kerrwood Lane**
**Cary North Carolina 27511(US)**
Inventor: **Shaver, Sammy Ray**
**103 Autumn Drive**
**Chapel Hill North Carolina 27514(US)**

(74) Representative: **Garrett, Michael et al**
**The Wellcome Research Laboratories Group Patents and Agreements Langley Court**
**Beckenham Kent BR3 3BS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 287 215 B1

Rank Xerox (UK) Business Services

## Description

The present invention relates to nucleoside derivatives which are useful in medical therapy particularly for the treatment or prophylaxis of certain viral infections more particularly human retroviral infections such as Acquired Immune Deficiency Syndrome (AIDS).

AIDS is an immunosuppressive or immunodestructive disease that predisposes subjects to fatal opportunistic infections. Characteristically, AIDS is associated with a progressive depletion of T-cells, especially the helper-inducer subset bearing the $OKT^4$ surface marker.

Human Immunodeficiency Virus (HIV) has been reproducibly isolated from patients with AIDS or with signs and symptoms that frequently precede AIDS. HIV is cytopathic and appears to preferentially infect and destroy $OKT^4$-bearing T-cells, and it is now generally recognised that HIV is the etiological agent of AIDS.

Since the discovery of HIV as the etiological agent of AIDS, numerous proposals have been made for anti-HIV chemotherapeutic agents that may be effective in the treatment of AIDS. Thus, for example, European Patent Specification No. 196185 describes 3′-azido-3′-deoxythymidine (which has the approved name zidovudine) and its pharmaceutically acceptable derivatives and their use in the treatment of human retrovirus infections including AIDS and associated clinical conditions. Zidovudine has been found to be of exceptional therapeutic benefit for the treatment of AIDS and associated conditions.

In the treatment of AIDS and related conditions, it is generally necessary to administer the anti-HIV chemotherapeutic agent on a regular basis to ensure the maintenance of sufficiently high antiviral levels of the drug in the patient, for example, zidovudine may need to be administered up to 6 times per day.

We have now discovered that a certain nucleoside derivative, namely 1-(3-azido-2,3-dideoxy-β-D-erythro-pentofuranosyl)-5-methyl-2(1H)-pyrimidinone, is capable of conversion in vivo to sidovudine when administered to a human or other animal subject. Such conversion is believed to be effected by the action such enzymes as xanthine oxidase/dehydrogenase or aldehyde oxidase present in the body of the subject to whom the said derivative is administered.

The above derivative can thus be used to improve the therapeutic efficacy of zidovudine by providing appropriate blood levels of the drug after administration of the above derivative.

According to one feature of the present invention we provide the compound of formula (I):

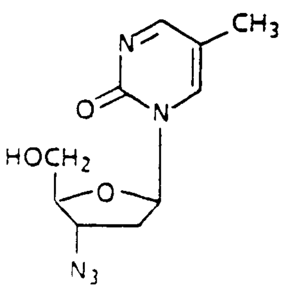

(I)

i.e. 1-(3-azido-2,3-dideox-β-D-erythro-pentofuranosyl)-5-methyl-2(1H)-pyrimidinone and its pharmaceutically acceptable salts, esters and salts of such esters. The compound of formula (I) and its above salts, esters and salts of esters are hereinafter referred to as the compounds according to the invention.

Preferred esters of the compound of formula (I) include carboxylic acid esters in which the non-carbonyl moiety of the ester grouping is selected from straight or branched chain alkyl, alkoxyalkyl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); and mono-, di- or triphosphate esters. Any reference to any of the above ester compounds also includes a reference to a pharmaceutically acceptable salt thereof.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

Examples of pharmaceutically acceptable salts of the compound of formula (I) and pharmaceutically acceptable esters thereof include base salts, e.g. derived from an appropriate base, such as alkali metal (e.g. sodium), alkaline earth metal (e.g. magnesium) salts, ammonium and $NX_4^+$ (wherein X is $C_{1-4}$ alkyl) and mineral acid salts, such as the hydrochloride.

According to further features of the present invention we provide :-

a) a compound according to the invention for use in medical therapy, especially in humans particularly for the treatment or prophylaxis of retroviral infections; and

b) use of a compound according to the invention in the manufacture of a medicament for the treatment or prophylaxis of retroviral infections.

Examples of retroviral infections which may be treated or prevented in accordance with the inventions include human retroviral infections such as Human Immunodeficiency Virus (HIV), HIV-2 and Human T-cell Lymphotropic Virus (HTLV) e.g. HTLV-I or HTLV-IV infections. The compounds according to the inventions are especially useful for the treatment or prophylaxis of AIDS and related clinical conditions such as AID-related complex (ARC), progressive generalised lymphadenopathy (PGL), AIDS-related neurological conditions and anti-HIV antibody-positive and HIV-positive conditions. The compounds may also be used in the treatment of prevention of psorriasis.

The compounds according to the invention, also referred to herein as the active ingredients, may be administered for therapy by any suitable route including oral, rectal, nasal, topical (including transdermal, buccal and sublingual), vaginal and parenteral (including subcutaneous, intramuscular, intravenous and intradermal). It will be appreciated that the preferred route will vary with the condition and age of the recipient and the nature of the infection, but oral or parenteral administration is generally preferred.

In general a suitable systemic dose of the compound 1-(3-azido-2,3-dideoxy-$\beta$-D-erythro-pentofuranosyl)-5-methyl-2(1H)-pyrimidinone will be in the range of 4.0 to 160 mg per kilogram body weight of the recipient per day, preferably in the range of 8 to 120 mg per kilogram body weight per day and most preferably in the range 20 to 80 mg per kilogram body weight per day to generate zidovudine to treat the mammal, e.g., human, who has an HIV infection. The desired dose is preferably presented as two, three, four, five, six or more sub-doses administered at appropriate intervals throughout the day. These sub-doses may be administered to unit dosage forms, for example, containing 10 to 1500 mg, preferably 20 to 1000 mg, and most preferably 50 to 700 mg of active ingredient per unit dosage form.

While it is possible for a compound according to the invention to be administered systemically (internally) alone it is preferable to present it as a pharmaceutical formulation. The formulations of the present invention comprise a compound according to the inention together with one or more acceptable carriers therefor and optionally other therapeutic agents. Each carrier must be "acceptable" in the sense of being compatible with the other ingredients of the formulation and not injurious to the patient. Formulations include those suitable for oral, rectal, nasal, topical (including transdermal, buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the compound according to the invention with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the compound of formula (I); as a powder or granules; as a solution or a suspension in an aqeuous or non-aqueous liquid; or as an oil-in-water liquid emulsion or a water-in-oil liquid emulsion. The active ingredient may also be presented as a bolus, electuary or paste. The compounds according to the invention may be formulated so as to provide slow or controlled release of the compound.

A tablet may be made by compression or moulding, optionally with one or more accessory ingredients. Compressed tablets may be prepared by compressing in a suitable machine the compound according to the invention in a free-flowing form such as a powder or granules, optionally mixed with a binder (e.g. povidone, gelatin, hydroxypropylmethylcellulose), lubricant, inert diluent, preservative, disintegrant (e.g. sodium starch glycollate, cross-linked povidone, cross-linked sodium carboxymethylcellulose) surface-active or dispersing agent. Moulded tablets may be made by moulding in a suitable machine a mixture of the powdered compound moistened with an inert liquid diluent. The tablets may optionally be coated or scored.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the compound according to the invention such carriers as are known in the art to be appropriate.

Formulations suitable for parenteral administration include aqueous and non-aqueous, isotonic, sterile, injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, the compound according to the invention.

The compounds according to the invention may also be presented for use in the form of veterinary formulations, which may be prepared, for example, by methods that are conventional in the art. Examples of such veterinary formulations include those adapted for :

a) oral administration, for example drenches (e.g. aqueous or non-aqueous solutions and suspensions); tablets or boluses; powders, granules or pellets for admixture with feed stuffs; pastes for application to the tongue;

b) parenteral administration for example by subcutaneous intramuscular or intravenous injection, e.g. as a sterile solution or suspension; or (when appropriate) by intramammary injection where a suspension or solution is introduced into the udder via the teat;

c) topical application, e.g. as a transdermal patch applied to the skin; or

d) intravaginally, e.g. as a pessary, cream or foam.

It will be appreciated that such formulations as are described above will also be suitable for the presentation of combinations according to the invention, whether unitary or separate formulations, and may be prepeared in a like manner.

It should be understood that in addition to the ingredients particularly mentioned above, the formulations of this invention may include other agents conventional in the art having regard to the type of formulation in question, for example, those suitable for oral administration may include such further agents as sweeteners, thickeners and flavoring agents.

The compound according to the invention may be employed in medical therapy in combination with other therapeutic agents, for example, acyclic nucleosides derivatives such as 9-(2-hydroxyethoxymethyl)-guanine, 2,3-dideoxynucleosides such as 2′,3′-dideoxycytidine, 2′3′-dideoxyadenosine and 2′,3′-dideoxyinosine, interferons such as alpha-interferon, nucleoside transport inhibitors such as dipyridamole, glucuronidation inhibitors such as probenicid, immunomodulators such as granulocyte macrophage colony stimulating factor (GMCSF) and other agents for example as described in European Patent Specification 217580. The component compounds of such combination therapy may be administered simultaneously, in either separate or combined formulations, or at different times e.g. sequentially such that a combined effect is achieved.

The compound of formula (I), and its pharmaceutically acceptable derivatives, may be prepared in conventional manner using techniques that are well known in the art, e.g. as described in Synthetic Procedures in Nucleic Acid Chemistry (1, 321 (1968)), T.A. Krenitsky et al (J. Med. Chem. 26, 891 (1983)); Nucleic Acid Chemistry, Improved and New Synthetic Processes, Methods and Techniques (Parts 1 and 2, Ed. L.D. Townsend, R.S. Tipson, (J. Wiley) 1978); J.R. Horwitz et al. (J. Org. Chem. 29, (July 1964) 2076-78); M. Imazawa et al. (J. Med. Chem., 45, 3274 (1980)); and R.P. Glinski et al. (J. Chem. Soc. Chem. Commun., 915 (1970)).

The present invention further includes a process for the preparation of the compound of formula (I) and its pharmaceutically acceptable salts, esters and salts of esters which comprises :

(A) reacting a compound of formula (II)

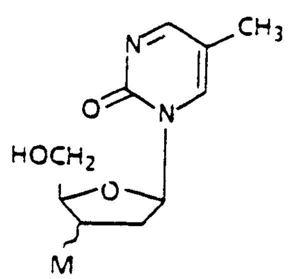

(II)

(wherein M represents a precursor group for the 3'-azido group) or a derivative (e.g. an ester) thereof, with an agent or under conditions serving to convert the said precursor group into the desired azido group;

(B) reacting a compound of formula (III)

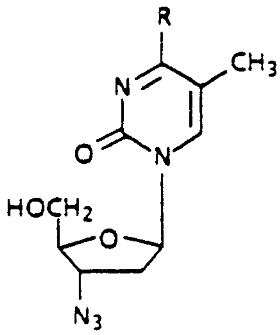

(III)

(wherein R represents a precursor group for a hydrogen atom) with an agent or under conditions serving to convert the said precursor group into a hydrogen atom; or

(C) reacting a compound of formula (IV)

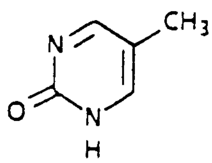

(IV)

or a functional equivalent thereof, with a compound serving to introduce the desired rebofuranosyl ring at the 1-position of the compound of formula (IV);

and thereafter, or simultaneously therewith, effecting at least one of the following conversions :

i) when a salt, ester or salt of an ester of a compound of formula (I) is formed, converting the said salt, ester or salt of an ester into the parent compound of formula (I); and

ii) when the compound of formula (I) is formed, converting the said compound into a pharmaceutically acceptable salt, ester or salt of an ester thereof.

In the above-described process according to the invention, it will be appreciated that the choice of the precursor compounds in processes (A) to (C) will be dictated largely by the particular compound that it is desired to prepare, the above-mentioned agents and conditions being selected accordingly from those that are known in the art of nucleoside synthetic chemistry. Examples of such conversion procedures are described hereinafter for guidance and it will be understood that they can be modified in conventional manner depending on the desired compound. In particular, for example, where a conversion is described which would otherwise result in the undesired reaction of labile groups then such groups may be protected in conventional manner, with subsequent removal of the protecting groups after completion of the conversion.

Thus, for example, with regard to process (A) the group M in the compound of formula (II) may represent, for example, a halogen (e.g. chlorine), hydroxy or organosulphonyloxy (e.g. trifluoromethylsulphonyloxy, methane sulphonyloxy or p-toluenesulphonyloxy) radical.

For the preparation of the compound of formula (I), a compound of formula (II) in which the group M is a halogen (e.g. chloro) group in the threo configuration (in which the 5'-hydroxy is advantageously protected, e.g. with a trityl group) may be treated for example with lithium or sodium azide. The 3'-threo-halogen (e.g. chlorine) starting material may be obtained, for example, by reaction of the corresponding 3'erythro-hydroxy compound with, for example, triphenylphosphine and carbon tetrachloride, or alternatively by treatment with organosulphonyl halide (e.g. trifluoromethanesulphonyl chloride) to form a corresponding 3'-erythro-organosulphonyloxy compound which is then halogenated, e.g. as described above. Alternatively a 3'-threo-hydroxy compound of formula (II) may be treated, for example with triphenylphosphine, carbon tetrabromide and lithium azide to form the corresponding 3'-erythro azido compound.

With regard to process (B), the precursor group R in formula (III) may for example, represent a 1,2,4-triazol-1-yl group, which may be converted to the desired compound of formula (I) for example, by treatment with hydrazine hydrate and silver oxide.

With regard to process (C), this may be effected for example by treating the appropriate pyrimidine of formula (IV) or a salt or protected derivative thereof, with a compound of formula :

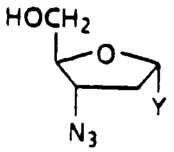

(wherein Y represents a leaving group, e.g. an acetoxy or benzoyloxy or halo (e.g. chloro) moiety, and the 5′-hydroxyl group is optionally protected, e.g. by a p-tolouyl group), and subsequently removing any protecting groups.

Where the compound of formula (I) is formed, such compound may be converted into a pharmaceutically acceptable phosphate or other ester by reacting the compound of formula (I) with respectively a phosphorylating agent, e.g. $POCl_3$ or an appropriate esterifying agent, e.g. an acid halide or anhydride. The compound of formula (I), including esters thereof, may be converted into pharmaceutically acceptable salt thereof in conventional manner, e.g. by treatment with an appropriate base.

Where a derivative of the compound of formula (I) is formed, for example an ester, such a derivative may be converted into the parent compound in conventional manner e.g. by hydrolysis.

The following Examples are intended for illustration only and are not intended to limit the scope of the invention in any way. The term 'Active Ingredient' as used in the examples means the compound of formula (I), i.e. 1-(3-azido-2,3-dideoxy-β-D-erythro-pentofuranosyl)-5-methyl-2(1H)-pyrimidinone.

Example 1 : Tablet Formulations

The following formulations A, B and C, are prepared by wet granulation of the ingredients with a solution of povidone, followed by addition of magnesium stearate and compression.

Formulation A

| | mg/tablet | mg/tablet |
|---|---|---|
| (a) Active Ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

Formulation B

| | mg/tablet | mg/tablet |
|---|---|---|
| (a) Active Ingredient | 250 | 250 |
| (b) Lactose | 150 | - |
| (c) Avicel pH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

Formulation C

| | mg/tablet |
|---|---|
| Active Ingredient | 100 |
| Lactose | 200 |
| Starch | 50 |
| Povidone | 5 |
| Magnesium Stearate | 4 |
| | 359 |

The following formulations, D and E, are prepared by direct compression of the admixed ingredients. The lactose used in formulation E is of the direct compression type (Dairy Crest - "Zeparox").

**Formulation D**

| | mg/tablet |
|---|---|
| Active Ingredient | 250 |
| Pregelatinized Starch NF15 | 150 |
| | 400 |

**Formulation E**

| | mg/tablet |
|---|---|
| Active Ingredient | 250 |
| Lactose | 150 |
| Avicel | 100 |
| | 500 |

Example 2 : Capsule Formulations

Formulation A

A capsule formulation is prepared by admixing the ingredients of Formulation D in Example 1 above and filling into a two-part hard gelatin capsule. Formulation B (infra) is prepared in a similar manner.

Formulation B

| | mg/capsule |
|---|---|
| Active Ingredient | 250 |
| Lactose B.P. | 143 |
| Sodium Starch Glycollate | 25 |
| Magnesium Stearate | 2 |
| | 420 |

Formulation C

| | mg/capsule |
|---|---|
| Active Ingredient | 250 |
| Macrogol 4000 B.P. | 350 |
| | 600 |

Capsules are prepared by melting the Macrogol 4000 B.P., dispersing the active ingredient in the melt and filling the melt into a two-part hard gelatin capsule.

Formulation D

| | mg/capsule |
|---|---|
| Active Ingredient | 250 |
| Lecithin | 100 |
| Arachis Oil | 100 |
| | 450 |

Capsules are prepared by dispersing the active ingredient in the lecithin and archis oil and filling the dispersion into soft, elastic gelatin capsules.

Example 3 : Injectable Formulation

Formulation A

| | | |
|---|---|---|
| Active Ingredient | | 0.200g |
| Hydrochloric Acid Solution 0.1 M | q.s. to pH | 4.0 to 7.0 |
| Sodium Hydroxide Solution 0.1 M | q.s. to pH | 4.0 to 7.0 |
| Sterile Water | q.s. to | 10 ml |

The active ingredient is dissolved in most of the water (35°-40°C) and the pH adjusted to between 4.0 and 7.0 with the hydrochloric acid or the sodium hydroxide as appropriate. The batch was then made up to volume with the water and filtered through a sterile micropore filter into a sterile 10 ml amber glass vial (type 1) and sealed with sterile closures and overseals.

Formulation B

| | |
|---|---|
| Active Ingredient | 0.125g |
| Sterile, Pyrogen-free, pH 7 Phosphate Buffer, q.s. to | 25ml |

Example 4 : Intramuscular Injection

| | | Weight (g) |
|---|---|---|
| Active Ingredient | | 0.20 |
| Benzyl Alcohol | | 0.10 |
| Glycofurol 75 | | 1.45 |
| Water for Injection | q.s. to | 3.00ml |

The active ingredient is dissolved in the glycofurol. The benzyl alcohol is then added and dissolved, and water added to 3ml. The mixture is then filtered through a sterile micropore filter and sealed in sterile 3ml amber glass vials (type 1).

Example 5 : Syrup

Formula A

| | | Weight (g) |
|---|---|---|
| Active Ingredient | | 0.2500 |
| Sorbitol Solution | | 1.5000 |
| Glycerol | | 2.0000 |
| Sodium Benzoate | | 0.0050 |
| Flavor, Peach 17.42.3169 | | 0.0125ml |
| Purified Water | q.s. to | 5.0000ml |

The active ingredient is dissolved in a mixture of the glycerol and most of the purified water. An aqueous solution of the sodium benzoate is then added to the solution, followed by addition of the sorbitol solution and finally the flavor. The volume is made up with purified water and mixed well.

Formulation B

| | | Weight (g) |
|---|---|---|
| Active Ingredient | | 0.250 |
| Sorbitol Solution | | 1.500 |
| Glycerol | | 0.005 |
| Dispersible Cellulose | | 0.005 |
| Sodium Benzoate | | 0.010ml |
| Flavour | q.s | |
| Purified Water q.s. to | | 5.000ml |

Mix the sorbitol solution, glycerol and part of the purified water. Dissolve the sodium benzoate in purified water and add the solution to the bulk. Add and disperse the dispersible cellulose and flavor. Add and disperse the active ingredient. Make up to volume with purified water.

Example 6 : 3′-Azido-3′-Deoxythymidine (AZT)

a) 2,3′-Anhydrothymidine

Thymidine (85.4g : 0.353mol) was dissolved in 500mL dry DMF and added to N-(2-chloro-1,1,2-trifluoroethyl)diethylamine (100.3g; 0.529mol) (prepared according to the method of D.E. Ayer, J.Med.Chem. 6, 608 (1963)). This solution was heated at 70 ° C for 30 minutes then poured into 950 mL ethanol (EtOH) with vigorous stirring. The product precipitated from this solution and was filtered. The EtOH supernatant was refrigerated then filtered to yield the title compound. mp. = 230 ° C.

b) 3′-Azido-3′-Deoxythymidine

2,3′-O-Anhydrothymidine (25g : 0.115mol) and $NaN_3$ (29g, 0.446mol) was suspended in a mixture of 250mL DMF and 38mL water. The reaction mixture was refluxed for 5 hours at which time it was poured into 1 litre of water. The aqueous solution was extracted with EtOAc (3 x 700 mL). The EtOAc extracts were dried over $Na_2SO_4$, filtered and the EtOAc was removed in vacuo to yield a viscous oil. This oil was stirred with 200mL water providing the title compound as solid which was collected by filtration. mp = 116-118 °C.

Example 7 : 5′-Acetyl-3′-azido-3′-deoxythymidine

To a solution of 3′-azido-3′-deoxythymidine (AZT) (20 g) in pyridine (50mL) at ambient temperature, acetyl chloride (2.1 equivalents) was added. The reaction was stirred for two hours and kept at 0 to 5 °C for 20 hours. It was poured onto ice water with stirring. The aqueous phase was decanted. The oily product was dissolved in ethyl acetate and extracted with water (5 times), 0.5N hydrochloric acid, water (2x), and dried over magnesium sulphate. The solution was filtered and evaporated in vacuo. The residual oil was dissolved in chloroform, applied to a silica gel column, and flash chromatographed using 2% methanol in chloroform. Fractions with product were evaporated and the oil was chromatographed again using ethyl acetate :hexane (6:4 v/v). Fractions with product were evaporated in vacuo to give 5′-acetyl-3′-azido-3′deoxythymidine as a white solid.
m.p. 96-98 °C

| | | | |
|---|---|---|---|
| Calculated | C, 46.60 | H, 4.89 | N, 22.65 |
| Found | C, 46.67 | H, 4.94 | N, 22.59 |

Example 8 : 1-(5-O-Acetyl-3-azido-2,3-dideoxy-β-D-erythro-pentofuranosyl)-5-methyl-4-(1,2,4-triazol-1-yl)-2-(1H)-pyrimidinone

5′-Acetyl-3′-azido-3′-deoxythymidine was reacted with 5 equivalents of 1,2,4-triazole and two equivalents of 4-chlorophenyl dichlorophosphate in dry pyridine at ambient temperature for 10 days. Silica gel chromatography of the crude product using 1:1 EtOAc/hexane (v/v) followed by combination and evaporation of the appropriate fractions yielded an oil.

Crystallization from EtOAc afforded the title compound as a solid 2.7g (7.5 mMol; 60%); m.p. = 143-145 °C. UV (nm) : at pH 1 λmax = 324,245,215 (ε = 9300, 10000, 20500), λmin = 282,233 (ε = 2100,8200); at pH 13 λmax = 276 (ε = 6000), λmin = 242 (ε = 2000), $H^1$NMR (DMSO-d6) 9.34, 8.40 (2s,2H triazolyl), 8.23 (s,1H,H6), 6.12 (t,1H,H1′,J = 6.16 Hz), 4.48-4.17 (m,4H,H3′,H4′,H5′), 2.35 (s,3H,5′-acetyl), 2.07 (s,3H,5CH3).

| Analysis for $C_{14}H_{16}N_{804}$ | | | |
|---|---|---|---|
| Calculated | C, 46.67 | H, 4.48 | N, 31.10 |
| Found | C, 46.58 | H, 4.51 | N, 31.02 |

Example 9 : 1-(3-Azido-2,3-dideoxy-β-D-erythro-pentofuranosyl)-5-methyl-(1H)-pyrimidinone

1-(5-O-Acetyl-3-azido-2,3-dideoxy-β-D-erythro-pentofuranosyl)-5-methyl-4-(1,2,4-triazol-1-yl)-2(1H)-pyrimidinone (0.5g, 1.4 mMol) was dissolved in $CH_3CN$ (10 mL) and treated with 85% hydrazine hydrate (0.105g, 2.1 mMol) for 30 minutes at ambient temperature, analogous to the procedure described by D. Cech and A. Holy, Coll.Czech.Chem.Comm. 42, 2246 (1977). The solvents were evaporated in vacuo and the residue chromatographed on silica gel with 9:1 $CHCl_3$/MeOH (v/v) as the eluting solvent. Collection and evaporation of appropriate fractions yielded a solid. The solid was dissolved in EtOH (50mL) containing $Ag_2O$ (0.35g, 1.5mMol, 1.5eq) and refluxed for 90 minutes. Filteration of the hot suspension through a bed of celite followed by removal of solvents in vacuo gave a solid. The solid was chromatographed on silica gel and eluted with 20:1 $CHCl_3$/MeOH (v/v). Combination and evaporation of appropriate fractions gave a solid which was dissolved in $NH_3$-saturated MeOH (50 mL) for 3 hours. Evaporation of the solvents in vacuo yielded an oil which was chromatographed on silica gel eluted with 20:1 $CHCl_3$MeOH (v/v). The appropriate fractions were combined and solvents evaporated in vacuo to give an oil which slowly solidified upon standing : mp = 62-63 °C; UV (nm) : at pH 1 λmax = 326, 212 (ε = 7700, 13000), λmin = 263 ( ε = 200);

at pH 13λ max = 322,218 ( ε= 22700, 10800), λmin = 246 (ε = 400); H' NMR: (DMSO- d6) 8.46 (d,1H,H4,J=3.28 Hz), 8.28 (d, 1H,H6, H=3.23 Hz), 6.00 (t,1H,H1',J=5.08 Hz), 5.31 (t, 1H, 5'OH, J=5.12 Hz), 4.4-4.3 (m, 1H, $H_3$'), 3.95-3.89 (m, 1H, $H_4$'), 3.8-3.6 (m, 2H, H5'), 2.5-2.3 (m, 2H, H2'), 2.03 (s, 3H, 5-$CH_3$).

Analysis calculated for $C_{10}H_{13}N_5O_3$.0.25 $H_2O$: C, 46.96; H, 5.32; N, 27.38.
Found : C, 47.05; H, 5.40; N, 27.14.

Example 10

1-(5-Acetyl-3-azido-2,3-dideoxy-β-D-erythro-pentafuranosyl)-5-methyl-2-pyrimidinone.

1-(5-Acetyl-3-azido-2,3-dideoxy-β-D-erythro-pentafuranosyl)-5-methyl-4-(1,2,4-triazol-1-yl)-2-(1H)-pyrimidinone (0.5 g, 1.4 mmol) was dissolved in $CH_3CN$ (10 ml) and treated with 85% hydrazine hydrate (0.105 g, 2.1 mmol) for 30 minutes at ambient temperature, analogous to the procedure described in D. Cech and A. Holy Coll. Czech. Chem. Comm., 42, 2246, (1977). The solvents were evaporated in vacuo and the residue chromatographed in silica gel with 9:1 $CHCl_3$ MeOH (v/v) as the eluting solvent. Collection and evaporation of appropriate fractions yielded a solid. The solid was dissolved in EtOH (50 ml) containing $Ag_2O$ (0.35 g, 1.5 mmol, 1.5 eq) and refluxed for 90 minutes. Filtration of the hot suspension through a bed of celite followed by removal of solvents in vacuo gave a solid. The solid was chromatographed on silica gel eluted with 20:1 $CHCl_3$/MeOH 9v/v). Combination and evaporation of appropriate fractions gave the title compound as a golden oil: UV(nm) at pH 1λmax=327 (ε=6900), λmin=254 (ε=700); at pH 13 λmax=322 (ε=17200), λmax=247 (ε=600); 1H NMR (DMSO-d6 δ) 8.46(d,J=3.13Hz, 1H, H4), 7.92-(d,J=3.90Hz,1H,H6), 6.03(dd,J=5.28Hz, 6.64Hz,1H,H1), 4.46-4.03(m,4H,H3,H4,H5), 2.71-2.32(m,2H,H2). 2.05,2.04(2s,6H,5-$CH_3$,acetyl).

| Analysis for $C_{12}H_{15}N_5O_4$ | | | |
|---|---|---|---|
| Calculated | C, 49.14 | H, 5.16 | N, 23.88 |
| Found | C, 49.39 | H, 5.22 | N, 23.75 |

Example 11

1-(3-Azido-2-deoxy-5-pivaloyl-β-D-ribofuranosyl)5-methyl-2-pyrimidinone

1-(3-Azido-2-deoxy-β-ribofuranosyl-5-methyl-2-pyrimidinone (0.4 g, 1.59 mmol) was dissolved in dry pyridine (10 ml) at 0°C under a nitrogen atmosphere. Pivaloyl chloride (0.588 ml, 4.78 mmol) was added with stirring and allowed to react for 72 hours. The reaction was quenched with ice then evaporated to dryness. The residue was chromatographed on silica gel eluted with 20:1 $CHCl_3$/MeOH (v/v) and the appropriate fractions combined and evaporated to give the title compound mp = 121-123°C; UV(nm) at pH 1 λ max=327(ε5900), λmin-269(ε=820); at pH 13 λmax-322 (ε=17200,λ min = 251 (ε =850): 1H NMR (DMSO-d6) δ8.48(d,J=3.12Hz,1H,H4), 7.88 (d,J=3.27Hz,1H,H6), 6.02(t,J=6.25Hz,1H,H1'), 4.48-4.39-(m,1H,H3'), 4.31-4.28(m,2H,H5'), 4.20-4.14(m,1H,H4'), 2.62-2.50(m,1H,H2), 2.41-2.27(m,1H,H2'), 2.04(s,3H,5-$CH_3$), 1.12(s,9H,-$C(CH_3)_3$).

| Analysis for $C_{15}H_{21}N_5O_4$ 0.1$H_2O$ | | | |
|---|---|---|---|
| Calculated | C, 53.42 | H, 6.34 | N, 20.77 |
| Found | C, 53.46 | H, 6.36 | N, 20.72 |

Example 12

1-[3-Azido-5-(3-chlorobenzoyl)-2-Deoxy-β-D-ribofuranosyl]-5-methyl-2-pyrimidinone

The title compounds was prepared from 3-chlorobenzoyl chloride in a manner analogous to Example 11 to yield a clear gum UV (nm) pH1 λmax=327( =6200), λmin=264( ε=1700), pH 13 λmax=318 (ε =8500),

λmin = 257(ε = 1600); 1H NMR(DMSO-d6), δ8.43(d,J = 1.27Hz,1H,H4), 7.93-7.52(m,5H,H6,benzoyl), 6.06-(t,J = 5,28Hz.1H,H1'), 4.67-4.51(m.3H,H3',H5'), 4.30(q.J = 4.25,1H,H4'), 2.59-2.39(m,2H,H2'), 1.85(s,3H,5-$CH_3$).

| Analysis for $C_{17}H_{16}N_5O_4Cl$ | | | |
|---|---|---|---|
| Calculated | C, 52.38 | H, 4.14 | N, 17.97 |
| Found | C, 52.52 | H, 4.19 | N, 17.82 |

Conversion of the Compound of Formula (I) to Zidovudine

In experiments in rats involving administration of the compound of formula (I) above in water by the oral route zidovudine was recovered in an average amount of 51%.

## Claims

**Claims for the following Contracting States : AT, BE, CH, DE, FR, GB, IT, LI, LU, NL, SE**

**1.** 1-(3-Azido-2,3-dideoxy-β-D-erythro-pentofuranosyl)-5-methyl-2(1H)-pyrimidinone and pharmaceutically acceptable salts, esters and salts of such esters thereof.

**2.** 1-(3-Azido-2,3-dideoxy-β-D-erythro-pentofuranosyl)-5-methyl-2(1H)-pyrimidinone.

**3.** Pharmaceutically acceptable salts and esters of 1-(3-azido-2,3-dideoxy- β-D-erythro-pentofuranosyl)-5-methyl-2(1H)pyrimidinone.

**4.** A compound as claimed in any of the preceding claims for use in medical therapy.

**5.** A compound as claimed in Claim 4 for use in the treatment or prophylaxis of human retroviral infections.

**6.** A compound as claimed in Claim 5 for use in the treatment or prophylaxis of Human Immunodeficiency Virus (HIV) infection.

**7.** A compound as claimed in Claim 5 for use in the treatment or prophylaxis of Acquired Immune Deficiency Syndrome (AIDS) or AIDS-related Complex (ARC).

**8.** Pharmaceutical formulations comprising at least one compound as claimed in any of claims 1 to 3 together with at least one pharmaceutical carrier or excipient.

**9.** Pharmaceutical formulations as claimed in claim 8 adapted for oral or parenteral administration.

**10.** Pharmaceutical formulations as claimed in claim 9 in the form of tablets or capsules.

**11.** A process for the preparation of 1-(3-azido-2,3-dideoxy-β-D-erythro-pentofuranosyl)-5-methyl-2(1H)-pyrimidinone and its pharmaceutically acceptable salts, esters and salts of esters which comprises:
(A) reacting a compound of formula (II)

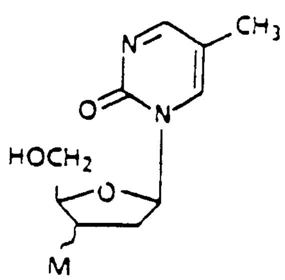

(II)

(wherein M represents a precursor group for the 3'-azido group) or a derivative thereof, with an agent or under conditions serving to convert the said precursor group into the desired azido group;
(B) reacting a compound of formula (III)

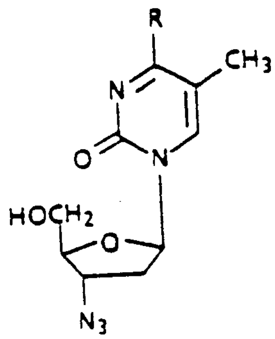

(III)

(wherein R represents a precursor group for a hydrogen atom) with an agent or under conditions serving to convert the said precursor group into a hydrogen atom; or
(C) reacting a compound of formula (IV)

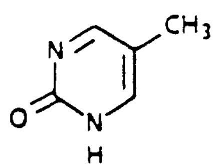

(IV)

or a functional equivelant thereof, with a compound serving to introduce the desired ribofuranosyl ring at the 1-position of the compound of formula (IV);
and thereafter, or simultaneously therewith, effecting at least one of the following conversions :
i) when a salt, ester or salt of an ester of the said pyrimidinone is formed, converting the said salt, ester or salt of an ester into the said pyrimidinone;
ii) when the said pyrimidinone is formed, converting the said pyrimidinone to a pharmaceutically acceptable salt, ester or salt of an ester thereof.

**Claims for the following Contracting States : ES, GR**

1. A process for the preparation of 1-(3-azido-2,3-dideoxy-β-D-erythro-pentofuranosyl)-5-methyl-2(1H)-pyrimidinone and its pharmaceutically acceptable salts, esters and salts of such esters, which comprises.
(A) reacting a compound of formula (II)

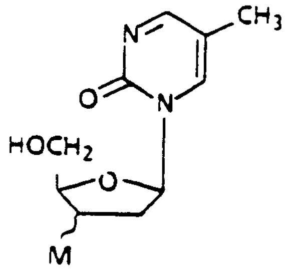

(II)

(wherein M represents a precursor group for the 3'-azido group) or a derivative thereof, with an agent or under conditions serving to convert the said precursor group into the desired azido group;
(B) reacting a compound of formula (III)

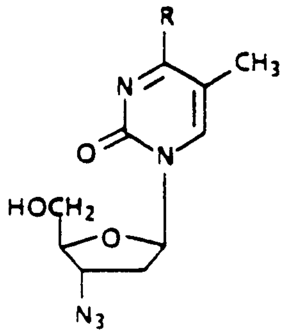

(III)

(wherein R represents a precursor group for a hydrogen atom) with an agent or under conditions serving to convert the said precursor group into a hydrogen atom; or

(C) reaction a compound of formula (IV)

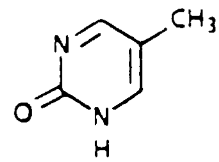

(IV)

or a functional equivalent thereof, with a compound serving to introduce the desired ribofuranosyl ring at the 1-position of the compound of formula (IV);

and thereafter, or simultaneously therewith, effecting at least one of the following conversions :

i) when a salt, ester or salt of an ester of the said pyrimidinone is formed, converting the said salt, ester or salt of an ester into the said pyrimidinone; and

ii) when the said pyrimidinone is formed, converting the said pyrmidinone to a pharmaceutically acceptable salt, ester or salt of an ester thereof.

**2.** A process as claimed in claim 1(B) in which a compound of formula (III) is employed in the form of a 5'-acetate derivative.

**3.** A process as claimed in claim 1(B) or claim 2 in which a compound of formula (III) is employed in which the precursor group R is a 1,2,4-triazol-1-yl group.

**4.** A process as claimed in claim 3 in which the said precursor group is converted to a hydrogen atom by treatment with hydrazine hydrate and silver oxide.

**Revendications**

**Revendications pour les Etats contractants suivants : AT, BE, CH, DE, FR, GB, IT, LI, LU, NL, SE**

**1.** La 1-(3-azido-2,3-didésoxy-$\beta$-D-érythropentofuranosyl)-5-méthyl-2(1H)-pyrimidinone et ses sels, esters et sels de ces esters pharmaceutiquement acceptables.

**2.** La 1-(3-azido-2,3-didésoxy-$\beta$-D-érythropentofuranosyl)-5-méthyl-2(1H)-pyrimidinone.

**3.** Sels et esters pharmaceutiquement acceptables de la 1-(3-azido-2,3-didésoxy-$\beta$-D-érythropentofuranosyl)-5-méthyl-2(1H)-pyrimidinone.

**4.** Composé suivant l'une quelconque des revendications précédentes, à utiliser en thérapeutique médicale.

**5.** Composé suivant la revendication 4, à utiliser dans le traitement ou la prophylaxie de l'infection rétrovirale chez l'être humain.

**6.** Composé suivant la revendication 5, à utiliser dans le traitement ou la prophylaxie d'une infection par le

virus de l'immunodéficience humaine (HIV).

**7.** Composé suivant la revendication 5, à utiliser dans le traitement ou la prophylaxie du syndrome de l'immunodéficience acquise (SIDA) ou du complexe apparenté au SIDA (ARC).

**8.** Compositions pharmaceutiques comprenant au moins un composé suivant l'une quelconque des revendications 1 à 3 avec au moins un véhicule ou excipient pharmaceutique.

**9.** Compositions pharmaceutiques suivant la revendication 8, adaptées à l'administration par voie orale ou parentérale.

**10.** Compositions pharmaceutiques suivant la revendication 9, sous la forme de comprimés ou de capsules.

**11.** Procédé de préparation de la 1-(3-azido-2,3-didésoxy-β-D-érythropentofuranosyl)-5-méthyl-2(1H)-pyrimidinone et de ses sels, esters et sels de ces esters pharmaceutiquement acceptables, qui comprend :
(A) la réaction d'un composé de formule (II) :

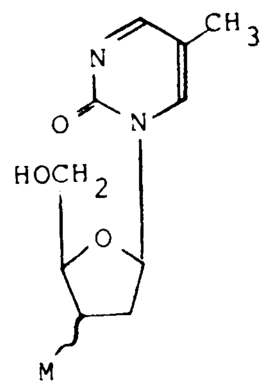

(II)

(où M représente un radical précurseur pour le radical 3'-azido) ou d'un dérivé (par exemple un ester) de celui-ci avec un agent ou dans des conditions servant à convertir le radical précurseur en le radical azido souhaité;
(B) la réaction d'un composé de formule (III) :

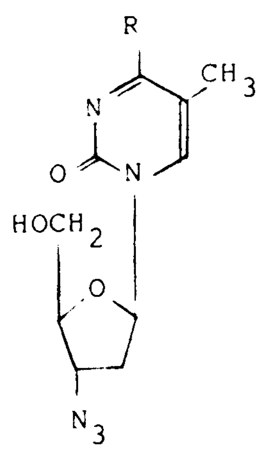

(III)

(où R représente un radical précurseur pour un atome d'hydrogène) avec un agent ou dans les conditions servant à convertir ce radical précurseur en un atome d'hydrogène, ou
(C) la réaction d'un composé de formule (IV) :

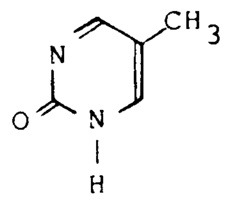

(IV)

ou d'un équivalent fonctionnel de celui-ci, avec un composé servant à introduire le cycle ribofuranosyle souhaité à la position 1 du composé de formule (IV);

et ensuite ou simultanément, l'exécution d'au moins l'une des conversions suivantes :

i) lorsqu'un sel, ester ou sel d'un ester de la pyrimidinone est formé, la conversion de ce sel, ester ou sel d'ester en la pyrimidinone, et

(ii) lorsque la pyrimidinone est formée, la conversion de la pyrimidinone en un sel, ester ou sel d'ester pharmaceutiquement acceptable de celle-ci.

**Revendications pour les Etats contractants suivants : ES, GR**

**1.** Procédé de préparation de la 1-(3-azido-2,3-didésoxy-β-D-érythropentofuranosyl)-5-méthyl-2(1H)-pyrimidinone et de ses sels, esters et sels de ces esters pharmaceutiquement acceptables, qui comprend :

(A) la réaction d'un composé de formule (II) :

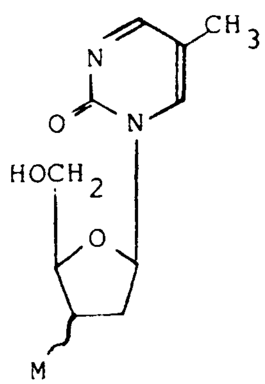

(II)

(où M représente un radical précurseur pour le radical 3'-azido) ou d'un dérivé (par exemple un ester) de celui-ci avec un agent ou dans des conditions servant à convertir le radical précurseur en le radical azido souhaité;

(B) la réaction d'un composé de formule (III) :

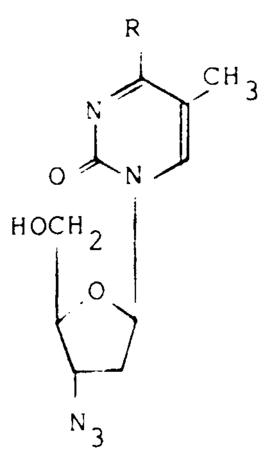

(III)

(où R représente un radical précurseur pour un atome d'hydrogène) avec un agent ou dans les conditions servant à convertir ce radical précurseur en un atome d'hydrogène, ou

(C) la réaction d'un composé de formule (IV) :

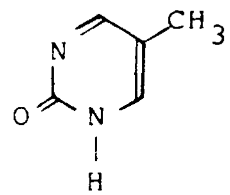

(IV)

ou d'un équivalent fonctionnel de celui-ci, avec un composé servant à introduire le cycle ribofuranosyle souhaité à la position 1 du composé de formule (IV);

et ensuite ou simultanément, l'exécution d'au moins l'une des conversions suivantes :

i) lorsqu'un sel, ester ou sel d'un ester de la pyrimidinone est formé, la conversion de ce sel, ester ou sel d'ester en la pyrimidinone, et

(ii) lorsque la pyrimidinone est formée, la conversion de la pyrimidinone en un sel, ester ou sel d'ester pharmaceutiquement acceptable de celle-ci.

**2.** Procédé suivant la revendication 1(B), dans lequel on utilise un composé de formule (III) sous la forme d'un 5'-acétate.

**3.** Procédé suivant la revendication 1(B) ou 2, dans lequel on utilise un composé de formule (III) dans lequel le radical R précurseur est un radical 1,2,4-triazol-1-yle.

**4.** Procédé suivant la revendication 3, dans lequel le radical précurseur est converti en un atome d'hydrogène par réaction avec l'hydrate d'hydrazine et l'oxyde d'argent.

**Patentansprüche**
**Patentansprüche für folgende Vertragsstaaten : AT, BE, CH, DE, FR, GB, IT, LI, LU, NL, SE**

**1.** 1-(3-Azido-2,3-didesoxy-β-D-erythropentofuranosyl)-5-methyl-2(1H)-pyrimidinon und pharmazeutisch annehmbare Salze, Ester und Salze derartiger Ester hievon.

**2.** 1-(3-Azido-2,3-didesoxy-β-D-erythropentofuranosyl)-5-methyl-2(1H)-pyrimidinon.

**3.** Pharmazeutisch annehmbare Salze und Ester von 1-(3-Azido-2,3-didesoxy-β-D-erythropentofuranosyl)-5-methyl-2(1H)-pyrimidinon.

**4.** Verbindung nach einem der vorhergehenden Ansprüche zur Verwendung in der medizinischen Therapie.

**5.** Verbindung nach Anspruch 4 zur Verwendung bei der Behandlung oder Prophylaxe von menschlichen Retrovirusinfektionen.

**6.** Verbindung nach Anspruch 5 zur Verwendung bei der Behandlung oder Prophylaxe von menschlicher Immundefektvirus (HIV)-Infektion.

**7.** Verbindung nach Anspruch 5 zur Verwendung bei der Behandlung oder Prophylaxe von erworbenem Immundefektsyndrom (AIDS) oder AIDS-verwandtem Komplex (ARC).

**8.** Pharmazeutische Formulierungen umfassend zumindest eine Verbindung nach einem der Ansprüche 1 bis 3 zusammen mit mindestens einem pharmazeutischen Träger oder Exzipienten.

**9.** Pharmazeutische Formulierungen nach Anspruch 8, die für orale oder parenterale Verabreichung geeignet sind.

10. Pharmazeutische Formulierung nach Anspruch 9 in Form von Tabletten oder Kapseln.

11. Verfahren zur Herstellung von 1-(3-Azido-2,3-didesoxy-$\beta$-D-erythropentofuranosyl)-5-methyl-2(1H)-pyrimidinon und dessen pharmazeutisch annehmbaren Salzen, Estern und Salzen von Estern, das umfaßt:
(A) das Umsetzen einer Verbindung der Formel (II)

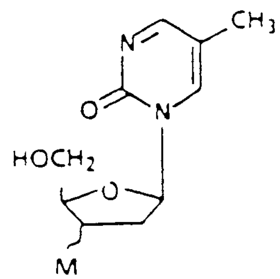

(II)

(worin M eine Vorläufergruppe für die 3'-Azidogruppe darstellt) oder eines Derivats hievon mit einem Mittel oder unter Bedingungen, das bzw. die zum Überführen dieser Vorläufergruppe in die gewünschte Azidogruppe dienen,
(B) das Umsetzen einer Verbindung der Formel (III)

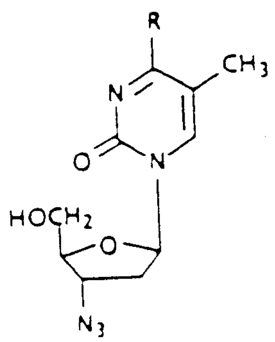

(III)

(worin R eine Vorläufergruppe für ein Wasserstoffatom darstellt) mit einem Mittel oder unter Bedingungen, das bzw. die zum Überführen dieser Vorläufergruppe in ein Wasserstofatom dienen, oder
(C) das Umsetzen einer Verbindung der Formel (IV)

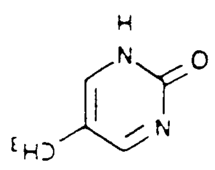

(IV)

oder eines funktionellen Äquivalents hievon mit einer Verbindung, die zum Einführen des gewünschten Ribofuranosylringes in Stellung 1 der Verbindung der Formel (IV) dient,
und anschließend oder gleichzeitig damit das Durchführen zumindest einer der folgenden Überführungen:
i) wenn ein Salz, Ester oder Salz eines Esters des Pyrimidinons gebildet wird, das Überführen des Salzes, Esters oder Salzes eines Esters in das Pyrimidinon,
ii) wenn das Pyrimidinon gebildet wird, das Überführen des Pyrimidinons in ein pharmazeutische annehmbares Salz, einen Ester oder das Salz eines Esters hievon.

**Patentansprüche für folgende Vertragsstaaten : ES, GR**

1. Verfahren zum Herstellen von 1-(3-Azido-2,3-didesoxy-$\beta$-D-erythropentofuranosyl)-5-methyl-2(1H)-pyrimidinon und pharmazeutisch annehmbaren Salzen, Estern und Salzen derartiger Ester hievon, das umfaßt:

(A) das Umsetzen einer Verbindung der Formel (II)

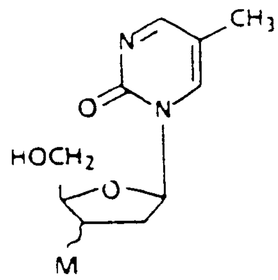

(II)

(worin M eine Vorläufergruppe für die 3'-Azidogruppe darstellt) oder eines Derivats hievon mit einem Mittel oder unter Bedingungen, das bzw. die zum Überführen dieser Vorläufergruppe in die gewünschte Azidogruppe dienen,

(B) das Umsetzen einer Verbindung der Formel (III)

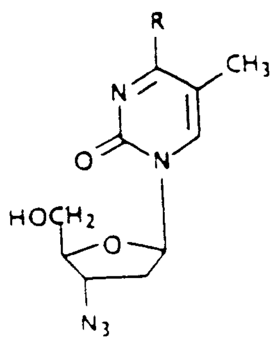

(III)

(worin R eine Vorläufergruppe für ein Wasserstoffatom darstellt) mit einem Mittel oder unter Bedingungen, das bzw. die zum Überführen dieser Vorläufergruppe in ein Wasserstofatom dienen, oder

(C) das Umsetzen einer Verbindung der Formel (IV)

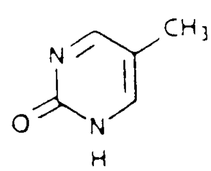

(IV)

oder eines funktionellen Äquivalents hievon mit einer Verbindung, die zum Einführen des gewünschten Ribofuranosylringes in Stellung 1 der Verbindung der Formel (IV) dient,

und anschließend oder gleichzeitig damit das Durchführen zumindest einer der folgenden Überführungen:

i) wenn ein Salz, Ester oder Salz eines Esters des Pyrimidinons gebildet wird, das Überführen des Salzes, Esters oder Salzes eines Esters in das Pyrimidinon,

ii) wenn das Pyrimidinon gebildet wird, das Überführen des Pyrimidinons in ein pharmazeutische annehmbares Salz, einen Ester oder das Salz eines Esters hievon.

2. Verfahren nach Anspruch 1(B), in dem eine Verbindung der Formel (III) in Form eines 5'-Acetatderivates verwendet wird.

3. Verfahren nach Anspruch 1(B) oder 2, in dem eine Verbindung der Formel (III) verwendet wird, in der die Vorläufergruppe R eine 1,2,4-Triazol-1-ylgruppe ist.

4. Verfahren nach Anspruch 3, in dem die Vorläufergruppe durch Behandlung mit Hydrazinhydrat und Silberoxid in ein Wasserstoffatom übergeführt wird.